(19) Europäisches Patentamt
European Patent Office
Office européen des brevets

(11) Publication number: 0 199 164 A1

# (12) EUROPEAN PATENT APPLICATION

(21) Application number: 86104642.3

(51) Int. Cl.4: G03C 7/34 , //C07C127/19

(22) Date of filing: 18.10.82

(43) Date of publication of application: 29.10.86 Bulletin 86/44

(60) Publication number of the original application in accordance with Art.76 EPC: 0 105 991

(84) Designated Contracting States: AT BE CH IT LI NL SE

(71) Applicant: KONISHIROKU PHOTO INDUSTRY CO. LTD.
No. 26-2, Nishishinjuku 1-chome Shinjuku-ku
Tokyo 160(JP)

(72) Inventor: Sato, Ryosuke
5995 Hino Hino-shi
Hino-shi Tokyo(JP)
Inventor: Kato, Katsunori
2-12-21 Nakanosanomachi
Hachioji-shi Tokyo(JP)
Inventor: Sasaki, Takashi
10-401 Takahatadai-Danchi 850 Misawa
Hino-shi Tokyo(JP)
Inventor: Sugita, Hiroshi
2-103 Famiiru-Nishihachioji 5-26 Sandamachi
Hachioji-shi Tokyo(JP)

(74) Representative: Ellis-Jones, Patrick George Armine et al
J.A. KEMP & CO. 14 South Square Gray's Inn
London WC1R 5EU(GB)

## (54) Silver halide photosensitive materials for color photography.

(57) A silver halide photosensitive material is disclosed for color photography which comprises a novel phenol type cyan coupler having the formula:

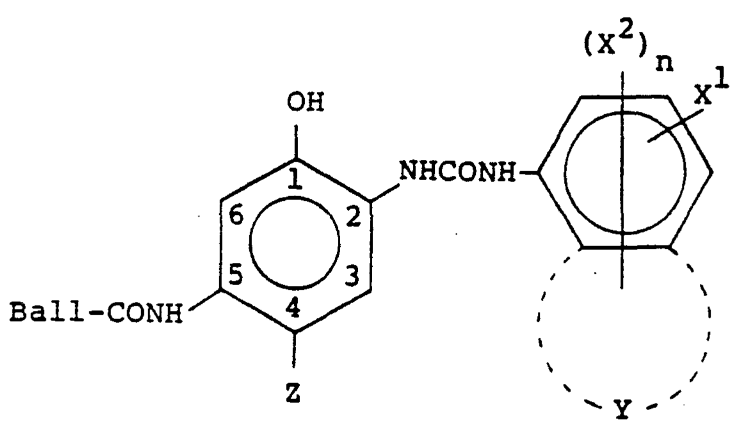

wherein $X^1$ is -CN and $X^2$, Y, Z and n have specific values.

The cyan coupler mentioned above is a novel cyan dye image-forming coupler and the photosensitive material containing the same according to the present invention has removed the various drawbacks in the prior art.

# SILVER HALIDE PHOTOSENSITIVE MATERIALS FOR COLOR PHOTOGRAPHY

This invention relates to a silver halide photosensitive material for color photography containing a novel cyan dye image-forming coupler.

A color image is usually formed by oxidative coupling of the oxidation product, which is formed by reduction of the exposed silver halide grains with an aromatic primary amine type color developing agent, with a coupler capable of forming yellow magenta or cyan dye in a silver halide emulsion.

As the coupler which is typically employed for forming the cyan dye, there may be mentioned phenols and naphthols. The basic properties required for the phenols used as couplers are that the dye obtained has good spectral absorption characteristics, i.e., the spectrum has no absorption in green region, but sharp absorption, that the dye formed shows sufficient fastness to light, heat, moisture and the like as well as good colorability, i.e., the dye shows sufficient color sensitivity and density and further that there is no loss of the dye even if the bleaching or bleach-fix bath used containing EDTA iron (III) complex as main ingredient becomes exhausted.

Moreover, it has been a serious problem in order to avoid environmental pollution to remove the benzyl alcohol incorporated into a color developing agent. However, the present situation is that a satisfactory color development can not be generally accomplished unless benzyl alcohol is added. A particularly remarkable reduction in color development can be seen with a phenol cyan coupler when benzyl alcohol is removed. From this point of view, there is a demand for a phenol cyan coupler with higher color development even if benzyl alcohol is not present.

In order to meet the aforesaid requirements, various studies have been made, but there has not yet been found a coupler capable of perfectly meeting all the foregoing properties. For instance, as disclosed in U.S. Patent No. 2,801,171, 6-[α-(2,4-di-tert amylphenoxy)butaneamido]-2,4-dichloro-3-methylphenol shows a good fastness to light, but has poor heat fastness, a great loss of the dye in an exhausted bleach-fix bath as well as a large color reliance on benzyl alcohol and difficult removal of benzyl alcohol from the color developing agent. U.S. Patent No. 2,895,826 discloses 2-heptafluorobutaneamido-5-[α-(2,4-di-tert-amylphenoxy)hexaneamido]phenol, which shows excellent heat fastness and lack of loss of dye in an exhausted bleach-fix bath, but has inferior light fastness and color development. The coupler disclosed in Japanese Patent Laid-open Application No. 53-10963 encounters problems about removal of benzyl alcohol and also light fastness. Further, those phenol type cyan couplers disclosed in U.S. Patent No. 3,839,044, Japanese Patent Laid-open Application No. 47-37425, Japanese Patent Publication No. 48-36894, Japanese Patent Laid-open Applications No. 50-10135, No. 50-117422, No. 50-130441, No. 50-108841 and No. 50-120334 are regarded as unsatisfactory with regard to heat fastness and removal of benzyl alcohol. The phenol couplers having a ureido group at the 2-position thereof disclosed in British Patent No. 1,011,940 and U.S. Patents No. 3,446,622, No. 3,996,253, No. 3,758,308, No. 3,880,661 and the like tend to form cyan dyes which show broad spectral absorption and, further, considerable absorption within the green region of the spectrum due to the maximum absorption in the red region or a relatively short wave range, which seems to be unfavourable in color reproduction. Other phenol couplers having a ureido group at the 2-position thereof as disclosed in Japanese Patent Laid-open Application No. 56-65134 (equivalent to European A-0028099) shows a considerably improved green absorption, but are still unsatisfactory in other respects.

We have made strenuous efforts to improve this situation and, as a result, have found that the couplers defined below substantially meet the above-recited characteristics required for a phenol cyan coupler.

More specifically, the silver halide photosensitive material for color photography according to the present invention is characterized by comprising a phenol type cyan coupler having the formula [I]

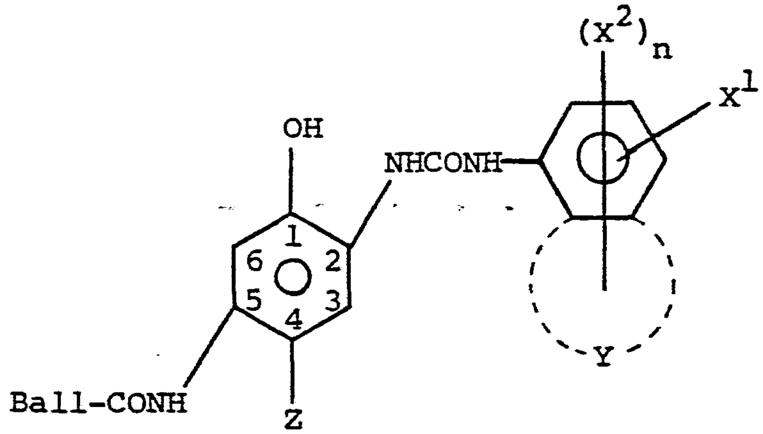

(I)

wherein

$X^1$ is -CN,

$X^2$ is a halogen atom, a hydroxy group, a nitro group or an optionally substituted alkyl, alkylcarbonyloxy, arylcarbonyloxy,. alkoxy, aryloxy, alkylcarbonyl or arylcarbonyl group; Y, when present, is a group of non-metallic atoms which completes a 5-or 6-membered condensed ring;

Z is a hydrogen atom or a group removable upon coupling with an oxidation product of a color developing agent; Ball is a ballast group; and

n is 0 or an integer from 1 to 4 inclusive such that, when n is 2 or more, each $X^2$ may be the same or different.

In the above formula, $X^2$ represents an optionally substituted alkyl group, an alkylcarbonyloxy or arylcarbonyloxy group, an alkoxy group, an alkylcarbonyl or arylcarbonyl group or an aryloxy group. As the alkyl group, there is preferably mentioned a $C_1$ to $C_5$ straight or branched alkyl group; illustrative examples thereof include methyl, ethyl, isopropyl, butyl, tert-pentyl, chloromethyl, acetonyl and phenethyl.

As the alkylcarbonyloxy or arylcarbonyloxy group, there may be mentioned a $C_1$ to $C_5$ alkylcarbonyloxy group and an arylcarbonyloxy group such as acetoxy, propionyloxy, pivaloyloxy, benzoyloxy, and naphthoyloxy.

As the alkoxy or aryloxy group, there is preferably mentioned a $C_1$ to $C_5$ alkoxy group and an aromatic alkoxy group; illustrative examples thereof include methoxy, tert-butoxy, ethoxy-methoxy, substituted or unsubstituted phenoxy.

As the alkylcarbonyl or arylcarbonyl group, there is preferably mentioned a $C_1$ to $C_5$ alkylcarbonyl group and an arylcarbonyl group such as acetyl, pivaloyl, acetoacetyl, benzoyl, naphthoyl and toluyl.

Also, the phenyl group of the phenylureido portion in the coupler may have a condensed ring formed by the group Y. Where the said phenyl group has the condensed ring, the substituent $X^2$ may be located on the phenyl moiety or the said condensed ring moiety. The condensed ring formed by the group Y may be, for example, naphthalene, quinoline, benzothiophene, benzofuran or isocoumaran.

As illustrative examples of the group Z which may be removed in a coupling reaction, there may be mentioned, for example, a halogen atom, e.g., chlorine, bromine or fluorine atom; an aryloxy group, a carbamoyloxy group, a carbamoylmethoxy group, an alkylcarbonyloxy or arylcarbonyloxy group, a sulfonamido group, a succinimido group, oxygen or nitrogen atom being attached directly to the coupling site in the said groups. Further examples thereof include those disclosed in U.S. Patent No. 3,471,563, Japanese Patent Laid-open Application No. 47-37425, Japanese Patent Publication No. 48-36894, Japanese Patent Laid-open Applications No. 50-10135, No. 50-117422, No. 50-130441, No. 51-108841, No. 50-120334, No. 52-18315, No. 53-52423 and No. 53-105226.

The ballasted acylamino group (Ball) substituted at the 5-position in the phenyl moiety acts as the "ballast" which can maintain the coupler in a specific layer so as to substantially prevent the said coupler dispersing to any other layer in a multi-layer color photographic element and should, therefore, possess sufficient "bulkiness" for such purposes. Illustrative examples thereof include an arylcarbonylamino group and an alkylcarbonylamino group.

In the case of the arylcarbonylamino group, the aromatic ring should have substituent(s) having a $C_5$ to $C_{18}$ alkyl chain. Such substituents having a $C_5$ to $C_{18}$ alkyl chain include, for example, an alkyl group, an alkylcarbonyl-or arylcarbonyl-oxy group, an alkylcarbonyl-or arylcarbonylamino group, a sul-

fonylamino group, an alkoxycarbonyl group, a carbamoyl group, a sulfamoyl group, an alkylamino group or a dialkylamino group. In the case of the alkylcarbonylamino group, there may be typically mentioned those wherein the aliphatic moiety is a $C_5$ to $C_{18}$ alkyl group, a phenoxyalkyl group or a phenylthioalkyl group. In the case of the phenoxyalkyl or phenylthioalkyl group, the phenoxy moiety may have a substituent having a $C_5$ to $C_{18}$ alkyl chain or the alkyl moiety may have 5 to 18 carbon atoms. As the ballasted alkylcarbonyl-or arylcarbonylamino group in the present coupler, Ball is particularly a phenoxyalkyl group or a phenylthioalkyl group. Illustrative examples of the alkylcarbonyl-or arylcarbonyl-amino group ballasted with a phenoxyalkyl group are recited below.

α-(3-Pentadecylphenoxy)butaneamido,

α-(2,4-di-tert-amylphenoxy)hexaneamido,

γ-(2,4-di-tert-amylphenoxy)butaneamido,

α-(2,4-di-tert-amylphenoxy)tetradecaneamido,

α-(4-butylsulfonylaminophenoxy)tetradecaneamido,

α-(4,-acetoxyphenoxy)dodecaneamido,

α-{p-[α-(4-hydroxyphenyl)-α,α-dimethyl]-tolyloxy}-dodecaneamido,

α-(4-carboxyphenoxy)dodecaneamido,

α-(2-chloro-4-butylsulfonylaminophenoxy)-tetradecaneamido,

α-(4-dimethylaminosulfonylaminophenoxy)-tetradecaneamido,

α-(3-dodecyloxyphenoxy)butaneamido,

α-(4-dodecyloxyphenoxy)butaneamido,

α-(4-hydroxyphenylthio)dodecaneamido, and

α-(4-acetylaminophenylthio)dodecaneamido.

Of the couplers which may be employed in this invention, there is preferably mentioned the coupler of the formula (I) wherein the phenylureido group substituted at the 2-position of the phenyl moiety has the following formula

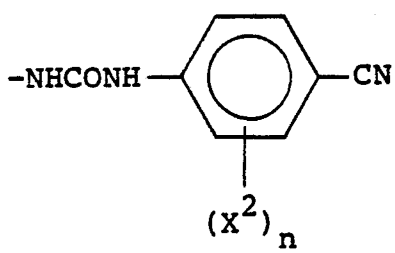

or

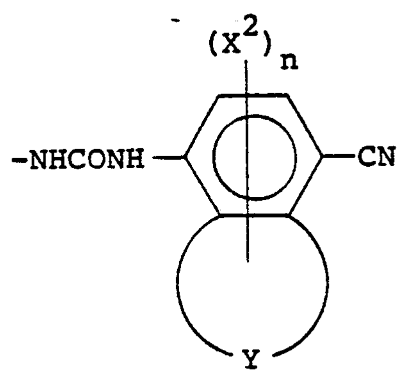

in which $X^2$, Y and n are as defined above.

Another preferable coupler of the present invention has the formula [II]

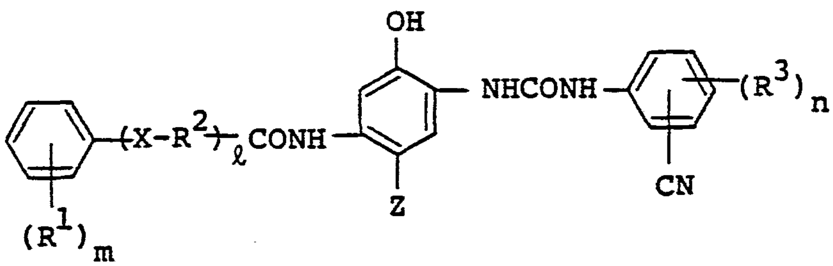

..... [II]

wherein X is an oxygen atom or a sulfur atom; $R^2$ is a, preferably $C_1$ to $C_{20}$, straight or branched alkylene group; the -CN group is located at the 2-or 3-position in the phenyl moiety to the ureido group; $R^3$ is a hydrogen atom, a halogen atom (preferably fluorine or bromine) or an optionally substituted alkyl (preferably, a $C_1$ -$C_4$ straight or branched alkyl group, particularly preferably methyl, tert-butyl), alkoxy (preferably, a $C_1$ to $C_8$ unsubstituted or substituted alkoxy group, particularly preferably methoxy, tert-butyloxy, methoxycarbonylmethoxy), aryloxy - (preferably, an unsubstituted or substituted phenoxy group), alkylcarbonyloxy or arylcarbonyloxy, alkylcarbonyl or arylcarbonyl (preferably, a $C_1$ to $C_8$ alkylcarbonyl group, particularly preferably an acetyl group or a pivaloyl group); $R^1$ is a hydrogen atom, a halogen atom (preferably, chlorine or bromine) a hydroxy group or an optionally substituted alkyl (preferably, a $C_1$ to $C_{20}$ straight or branched alkyl group, particularly preferably methyl, tert-butyl, tert-pentyl, tert-octyl, dodecyl or pentadecyl), aryl (preferably, phenyl), heterocyclic (preferably, an N-containing heterocyclic group), aralkyl (preferably, benzyl or phenethyl), alkoxy - (preferably, a $C_1$ to $C_{20}$ straight or branched alkoxy group, particularly preferably methoxy, ethoxy, tert-butoxy, octyloxy, decyloxy, dodecyloxy), aryloxy - (preferably, phenoxy), alkylcarbonyloxy or arylcarbonyloxy group (preferably acetoxy or benzoyloxy), carboxy, alkoxycarbonyl (preferably, a $C_1$ to $C_{20}$ straight or branched, unsubstituted or substituted alkyloxycarbonyl group), aryloxycarbonyl (preferably, an unsubstituted or substituted phenoxycarbonyl group), mercapto, an alkylthio group - (preferably, a $C_1$ to $C_{20}$ straight or branched, unsubstituted or substituted alkylthio group), arylthio - (preferably, an unsubstituted or substituted phenylthio group), alkylsulfonyl (preferably, a $C_1$ -$C_{20}$ straight or branched alkylsulfonyl group), arylsulfonyl (preferably, an unsubstituted or substituted benzenesulfonyl group), alkylcarbonyl or arylcarbonyl (preferably, a $C_1$ to $C_{20}$ straight or branched alkylcarbonyl group or an unsubstituted or substituted benzenesulfonyl group), alkylcarbonyl-or arylcarbonyl-amino group (preferably, a $C_1$ to $C_{20}$ straight or branched alkylcarbonamido group or an unsubstituted or substituted benzenecarbonamido group), sulfonamido (preferably, a $C_1$ to $C_{20}$ straight or branched, unsubstituted or substituted alkylsulfonamido group or an unsubstituted or substituted benzenesulfonamido group), carbamoyl (preferably, a $C_1$ to $C_{20}$ straight or branched alkylaminocarbonyl group or an unsubstituted or substituted phenylaminocarbonyl group) or sulfamoyl group - (preferably, a $C_1$ to $C_{30}$ straight or branched alkylaminosulfonyl group or an unsubstituted or substituted phenylaminosulfonyl group);

n is 0 or an integer of 1-3; m is an integer of 1 to 4; and ℓ is 0 or 1; and Z is as defined in the above formula [I].

Illustrative examples of the coupler, which may be employed in the photosensitive material for color photography according to this invention, are given hereinbelow.

(1)

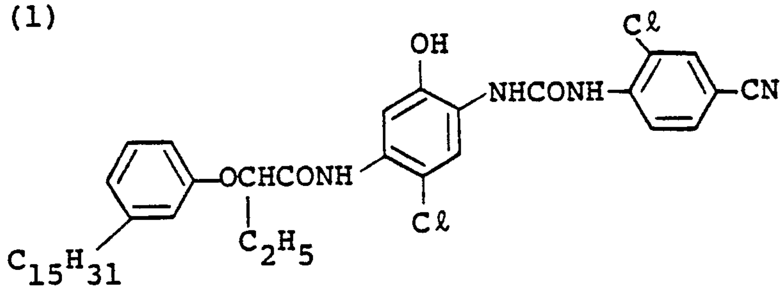

(2)

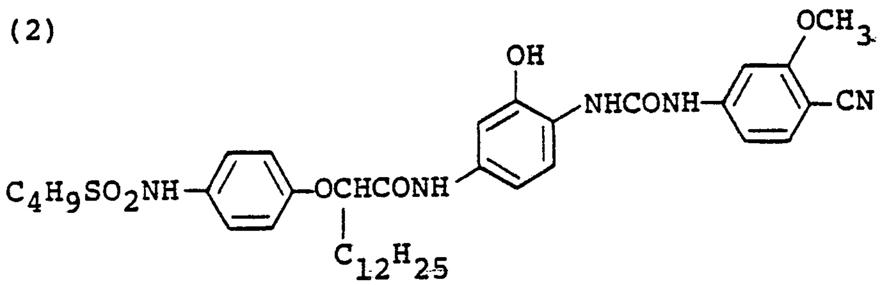

(3)

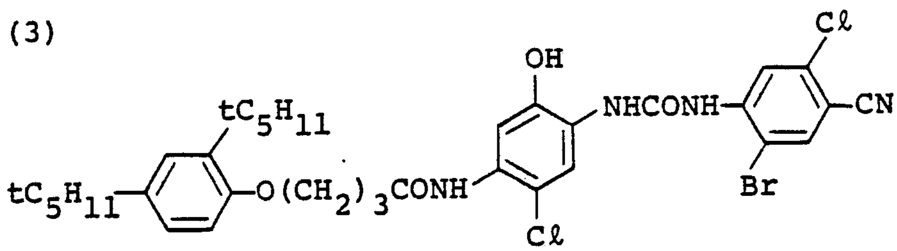

(4)

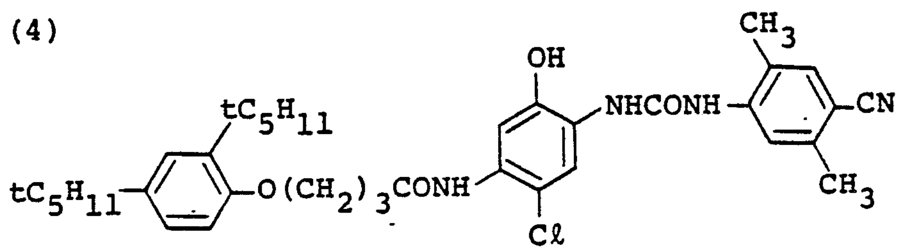

(5)

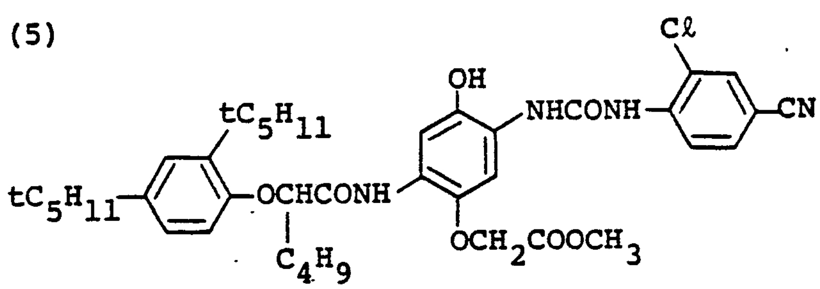

(6)

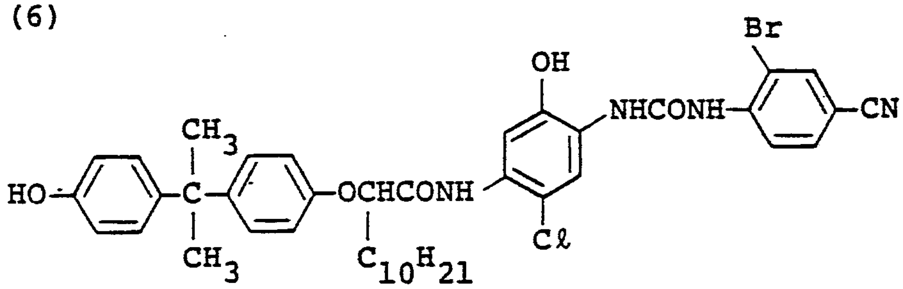

(7)

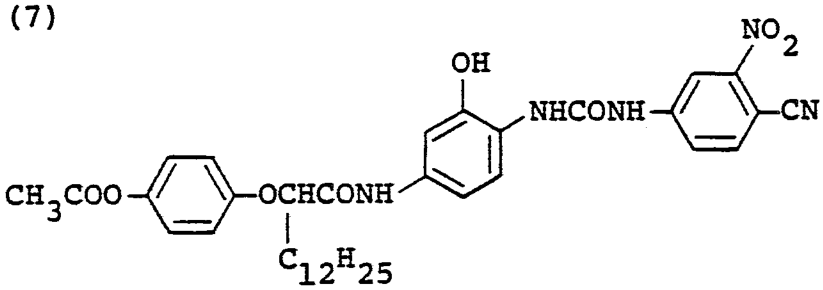

(8)

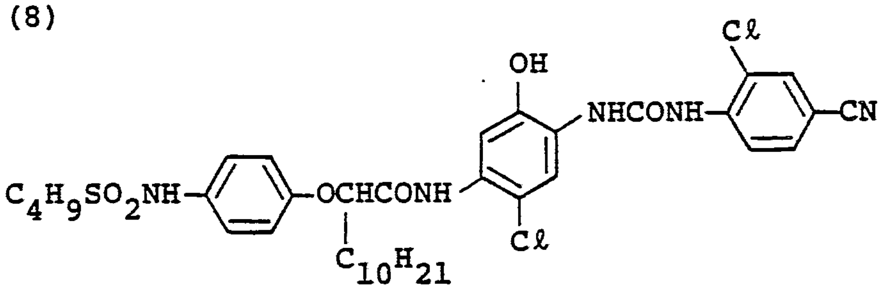

(9)

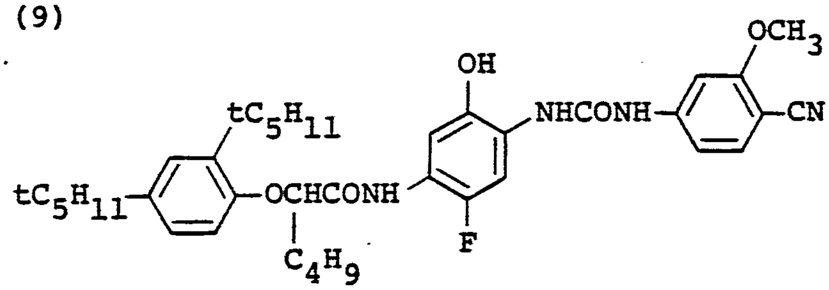

(10)

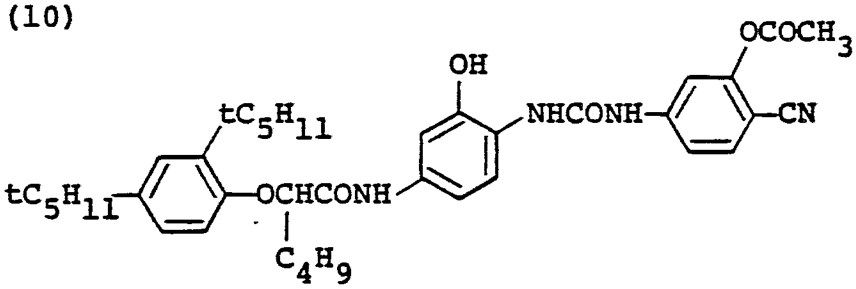

(11)

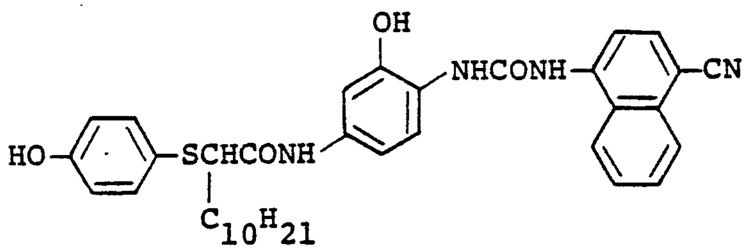

(12)

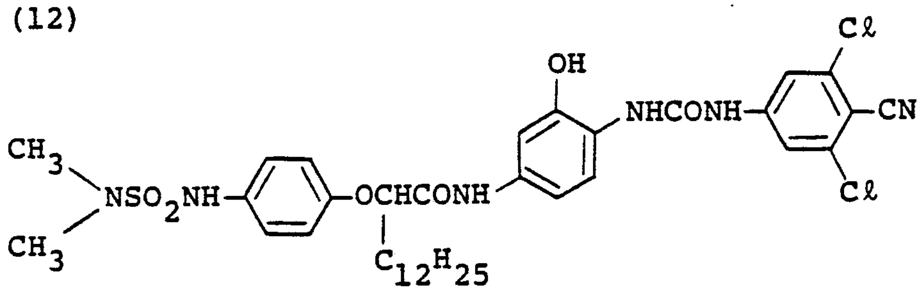

(13)

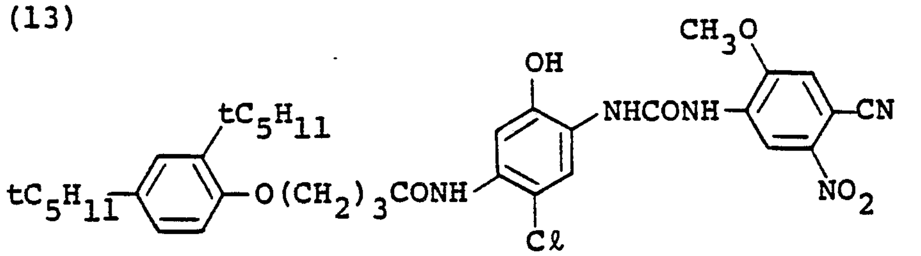

(14)

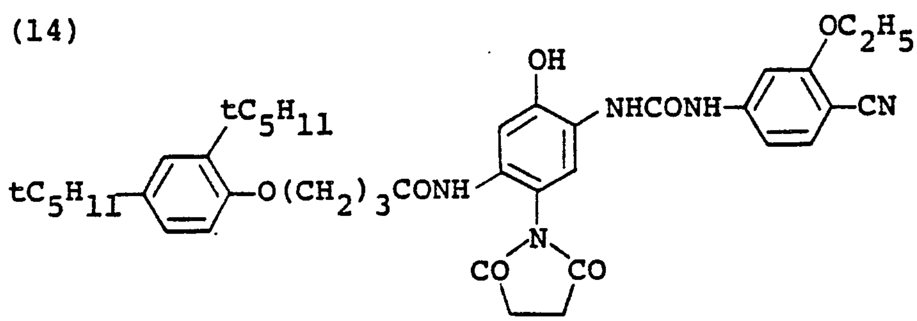

(15)

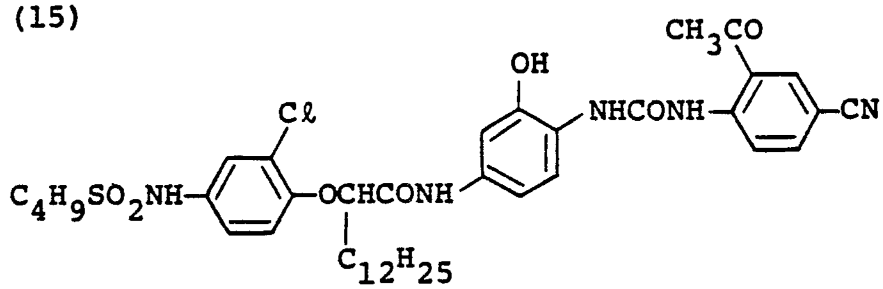

(16)

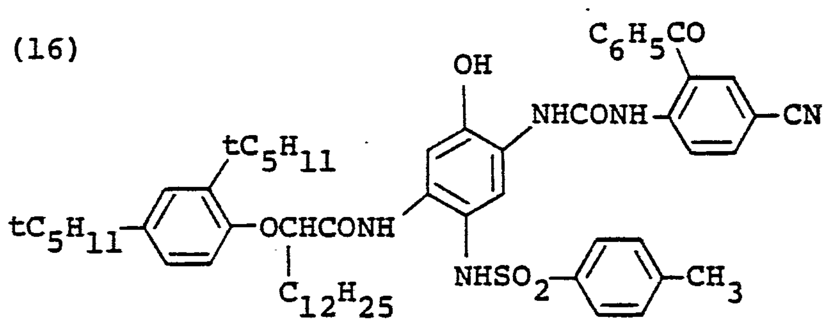

(17)

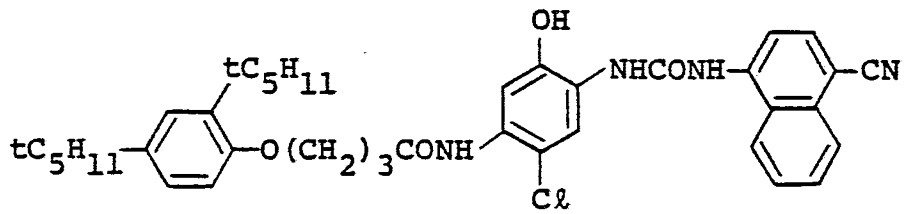

(18)

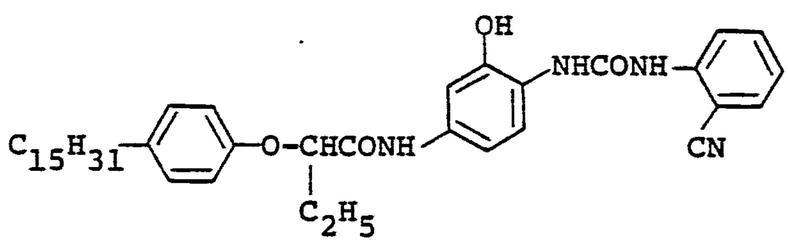

(19)

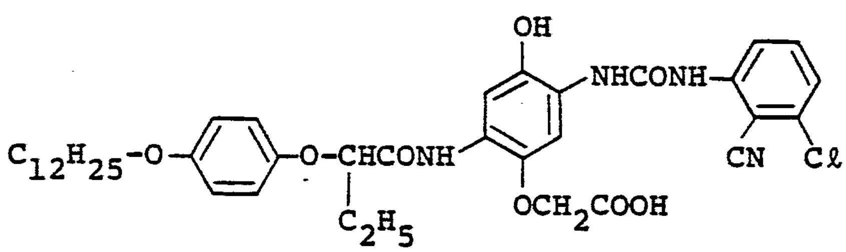

(20)

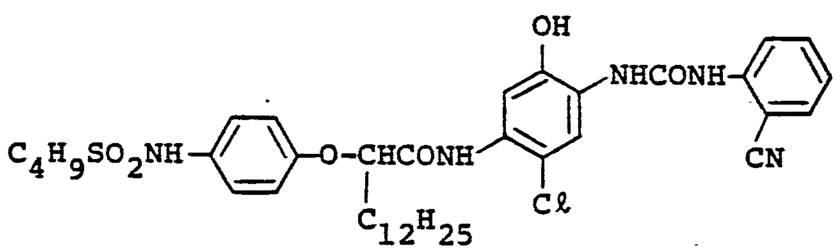

(21)

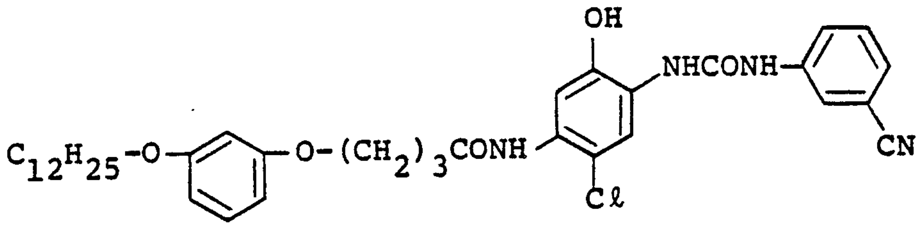

(22)

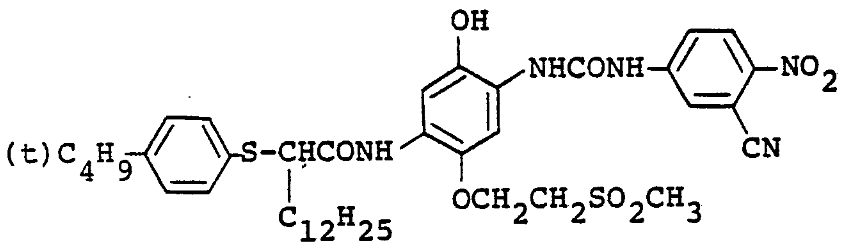

(23)

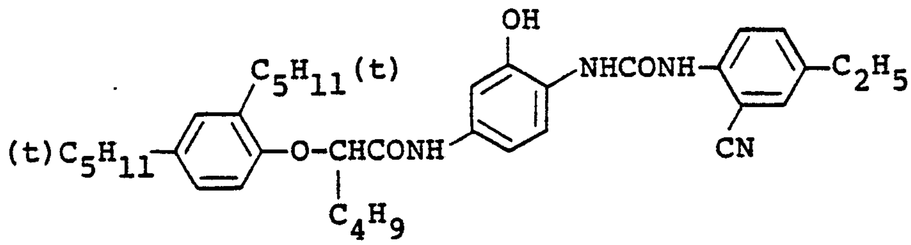

(24)

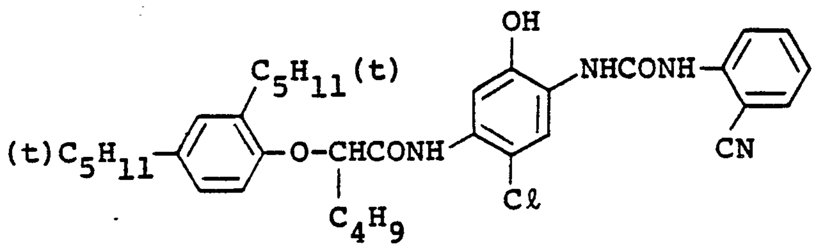

(25)

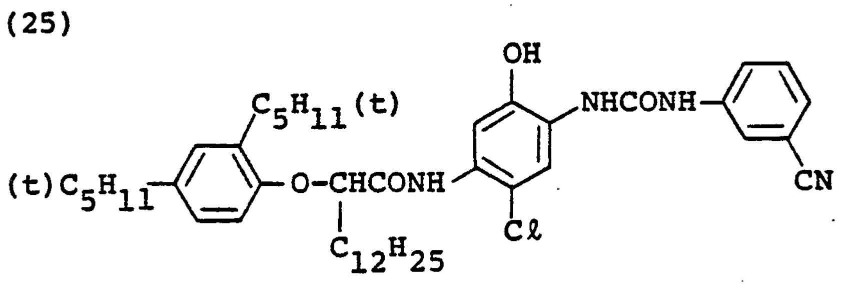

(26)

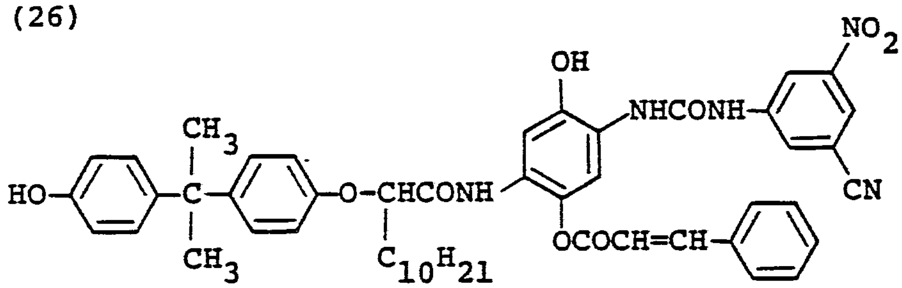

(27)

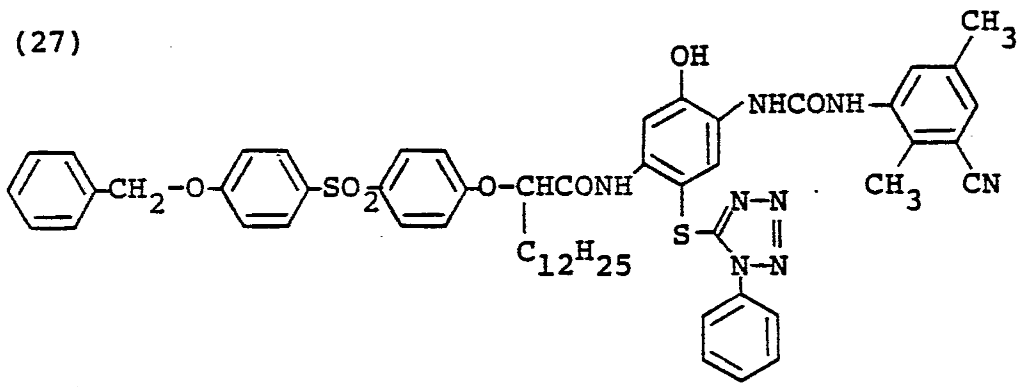

(28)

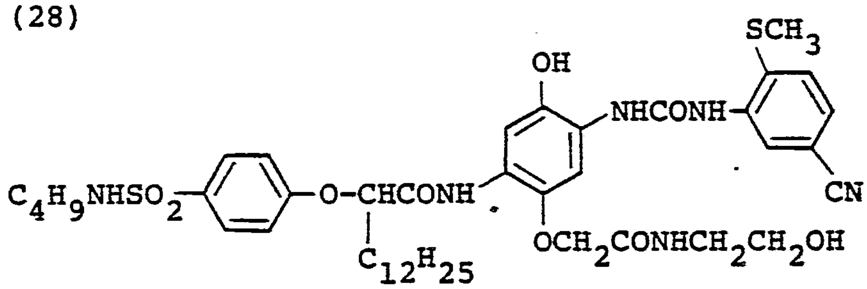

(29)

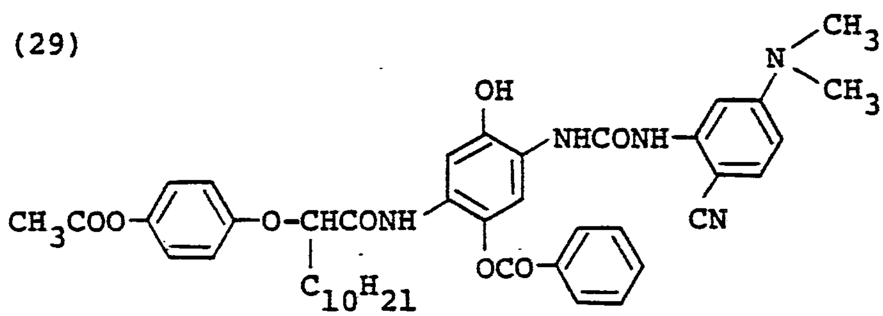

(30)

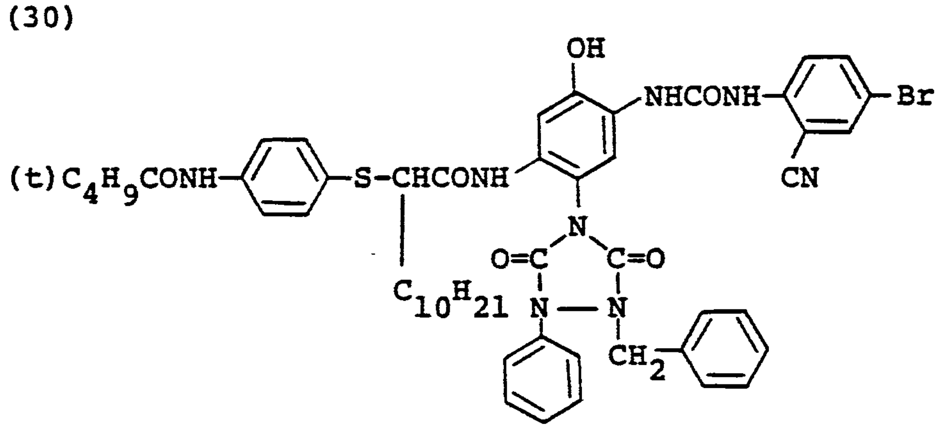

(31)

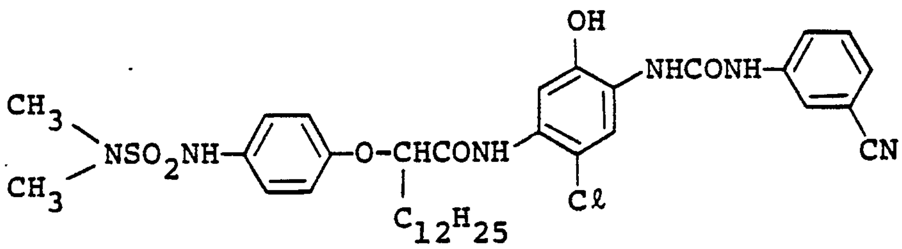

(32)

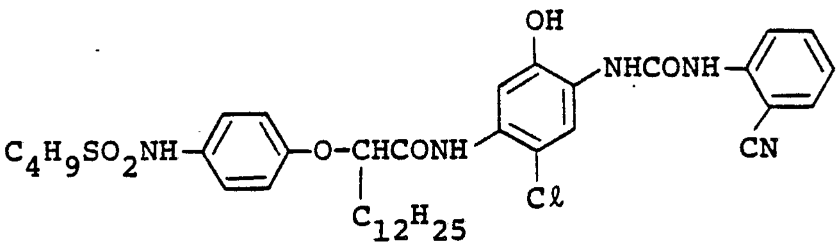

(33)

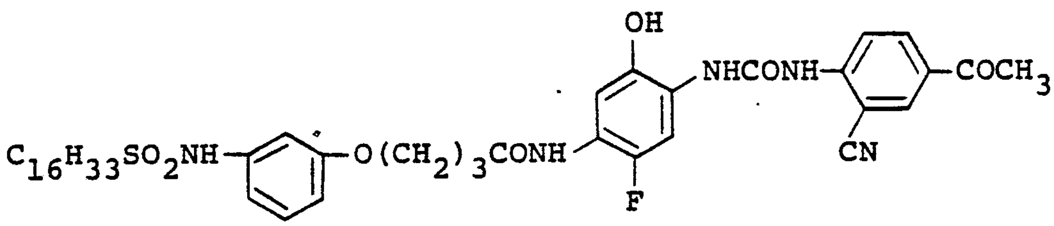

(34)

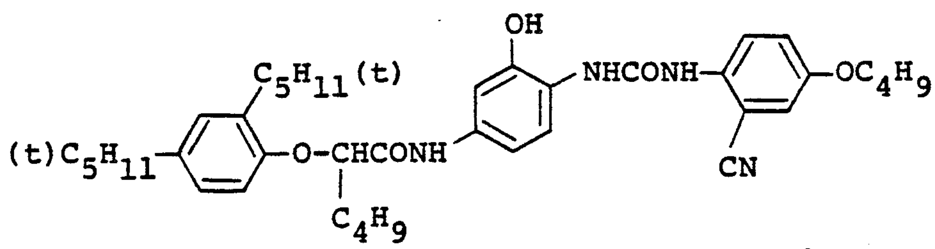

(35)

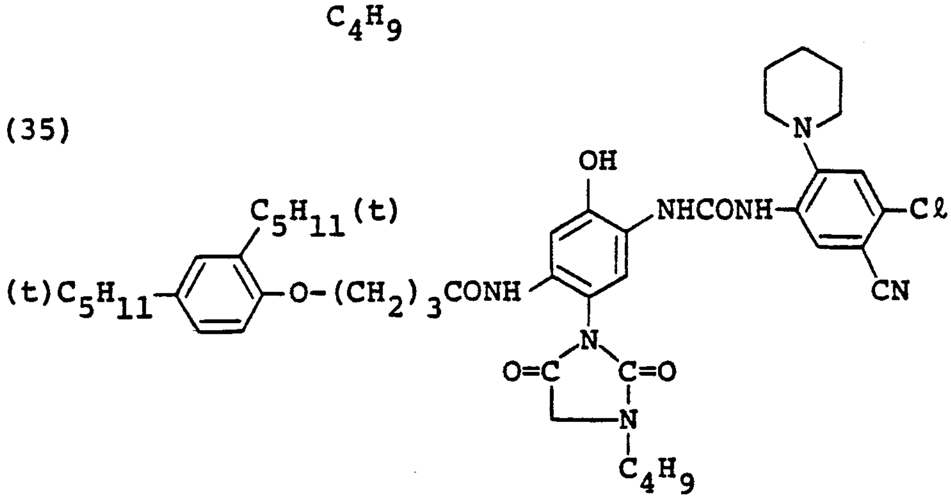

(36)

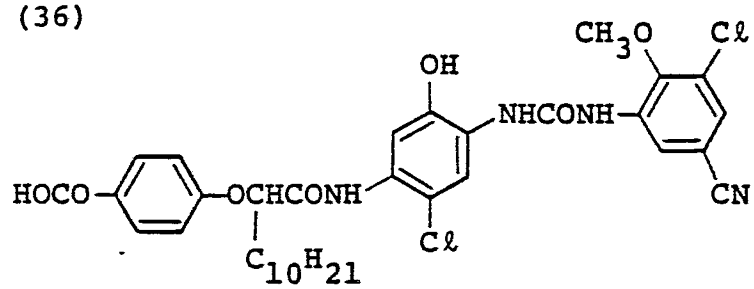

(37)

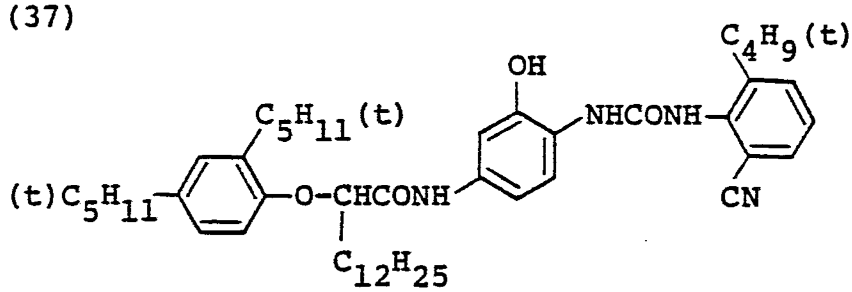

(38)

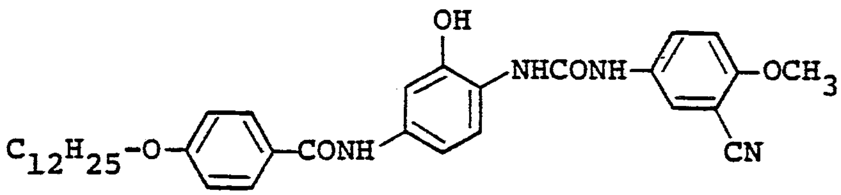

(39)

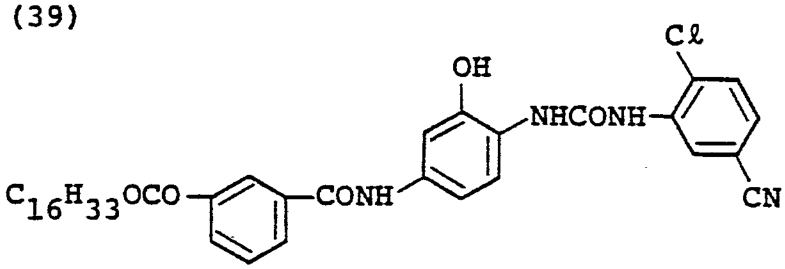

(40)

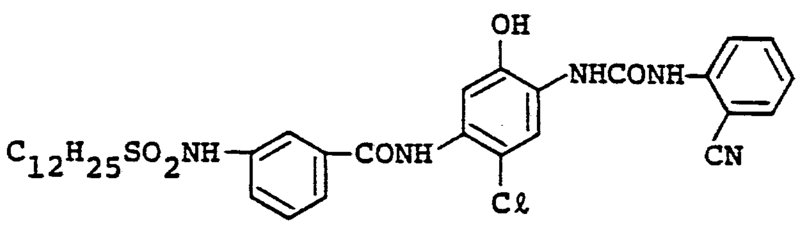

(41)

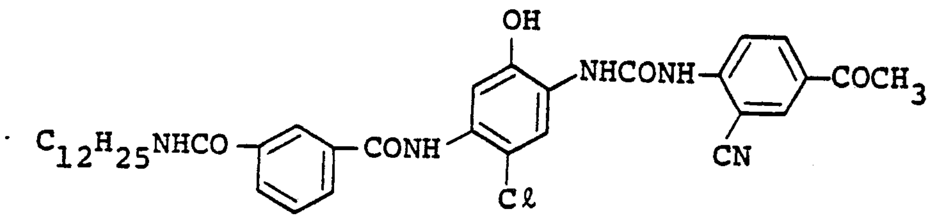

(42)

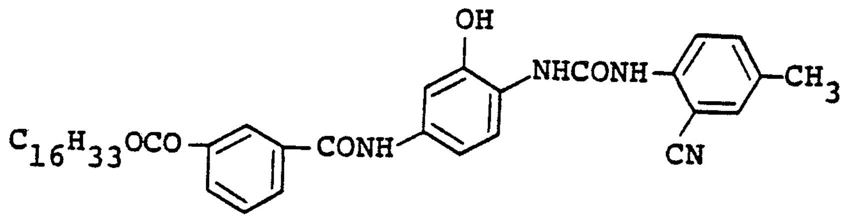

(71)

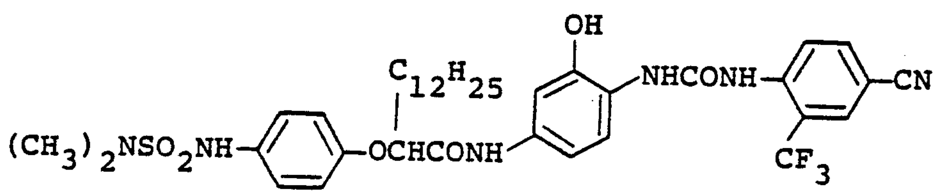

(73)

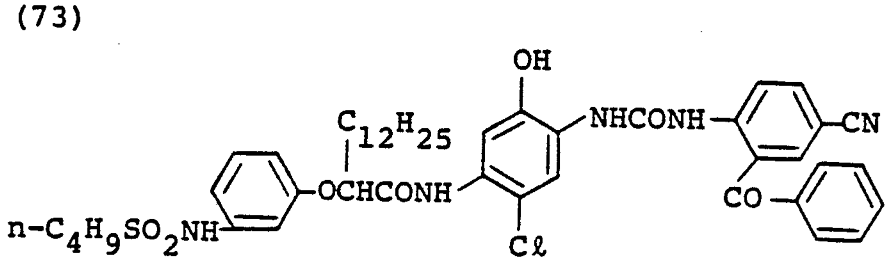

(76)

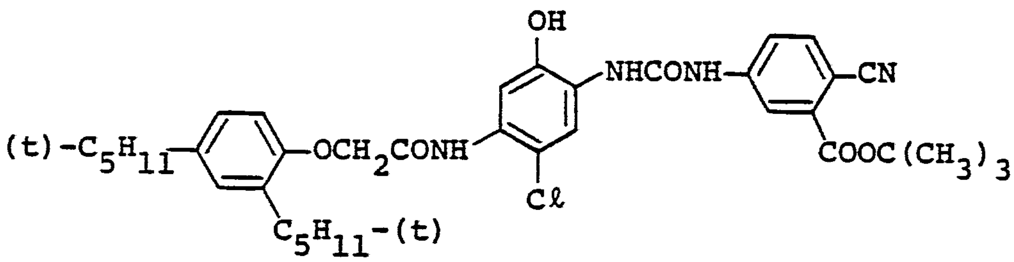

(78)

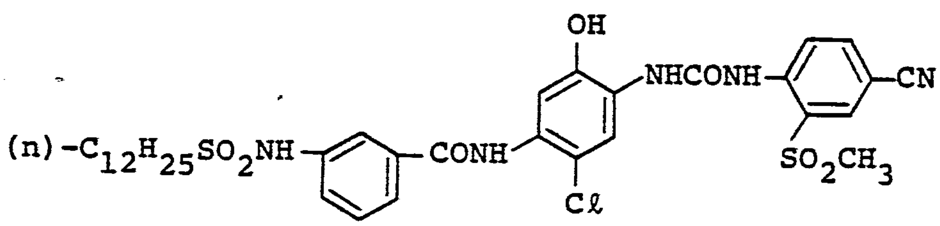

The couplers which may be employed in this invention can be prepared by reacting a substituted p-cyanophenyl isocyanate with a suitable aminophenol, for example, 2-amino-5-nitrophenol or 2-amino-4-chloro-5-nitrophenol to produce a 2-(substituted p-cyanophenyl)ureido compound. Then, the nitro group of the latter compound is reduced to the amino group in a conventional manner and the ballast group is attached to the said amino group to produce the desired coupler.

Representative synthesis examples of the present coupler are given below.

Synthesis Example 1

Coupler No. 2

(A) Synthesis of 2-(p-cyano-m-methoxyphenyl) ureido-5-nitrophenol

A suspension of 15.4 g. of 2-amino-5-nitrophenol in 300 mℓ. of toluene was refluxed. A solution of 17.4 g. of p-cyano-m-methoxyphenyl isocyanate in 150 mℓ. of toluene was added dropwise thereto. After completion of the dropwise addition, the resulting mixture was heated under reflux for 1 hour and then cooled. The precipitate was filtered and washed successively with hot toluene and alcohol to give 22 g. of the end product.

(B) Synthesis of Coupler No. 2

The nitrophenol produced in (A) (6.6 g.) was subjected to hydrogenation at ordinary temperature and atmospheric pressure in 200 mℓ. of alcohol using palladium-carbon catalyst. After the catalyst was filtered off, the filtrate was concentrated and the residue was dissolved in 200 mℓ. of acetonitrile. To the resulting solution were added 1.6 g. of pyridine and then 9.5 g.. of 2-(4-butylsulfonylaminophenoxy)tetradecanoyl chloride under stirring at room temperature. After stirring was continued at room temperature for 5 hours, the reaction mixture was poured into water, extracted with ethyl acetate. The ethyl acetate was distilled off and the residue crystallized from methanol and then acetonitrile to give 7.2 g. of the end product, which was identified by mass spectrum and NMR spectrum.

Synthesis Example 2

Coupler No. 1

(A) Synthesis of 2-(o-chloro-p-cyanophenyl) ureido-4-chloro-5-nitrophenol

A mixture of 18.8 g. of 2-amino-4-chloro-5-nitrophenol, 27.3 g. of phenyl o-chloro-p-cyanophenyl-carbamate and 0.6 g. of imidazole in 400 mℓ. of xylene was heated under reflux for 5 hours. After cooling, the precipitate was filtered off and the residue was washed with xylene and then alcohol to give 23.5 g. of the end product.

(B) Synthesis of Coupler No. 1

The nitrophenol produced in (A) (11 g.) was subjected to hydrogenation at ordinary temperature and atmospheric pressure in 300 mℓ. of alcohol using palladium-carbon catalyst. After completion of the reaction, the catalyst was filtered off, the filtrate was concentrated and to the residue were added 250 mℓ. of acetonitrile. To the resulting mixture were added 2.4 g. of pyridine and then 12.3 g. of 2-(3-pentadecylphenol)butanoyl chloride under stirring at room temperature. After stirring at room temperature for 6 hours, the reaction mixture was poured into water and extracted with ethyl acetate. The ethyl acetate was distilled off and the residue was chromatographed over silica gel using as eluant benzene-ethyl acetate. The fractions containing the end product were combined and the solvent was distilled off. The residue was recrystallized from methanol to give 5.8 g. of the end product, which was identified by mass spectrum and NMR spectrum.

Synthesis Example 3.

Coupler No. 24

Synthesis of 2-(o-cyanophenyl)ureido-4-chloro-5-{α-(2,4-di-tert-pentylphenoxyl)hexaneamido}phenol

To a suspension of 18.9 g. of 2-amino-4-chloro-5-nitrophenol in 200 mℓ. of toluene was added a solution of 16 g. of o-cyanophenyl isocyanate in 100 mℓ. of toluene under stirring at room temperature. The resulting mixture was boiled under reflux for 1 hour and then allowed to cool to room temperature. The crystalline substance thus separated was filtered, washed with hot toluene and then cold methanol to give 31 g. of the product with mp 251 - 255°C.

A suspension of 3.3 g. of the so obtained 2-(o-cyanophenyl)ureido-4-chloro-5-nitrophenol in 200 mℓ. of tetrahydrofuran was subjected to catalytic reduction using palladium-carbon catalyst. After a theoretical volume of hydrogen was absorbed, 0.9 mℓ. of pyridine was added to the reaction mixture and then a solution of 3.7 g. of 2-(2,4-di-tert-pentylphenoxy)hexanoyl chloride in 50 mℓ. of tetrahydrofuran was added thereto under stirring at room temperature. After completion of the addition, the reaction was continued for further 1 hour and the catalyst was filtered off. The filtrate was added to ice-water containing 10 mℓ. of conc. hydrochloric acid and extracted with ethyl acetate. The extract was washed with water, dried over sodium sulfate and concentrated under reduced pressure to leave an oily substance. The substance was solidified with a mixture of benzene with hexane to give 2.6 g. of the end product as a white solid with mp 163-168°C.

Analysis (%)

| | C | H | N | Cℓ |
|---|---|---|---|---|
| Calc'd: | 68.28 | 7.16 | 8.85 | 5.60 |
| Found : | 67.95 | 6.88 | 9.03 | 5.82 |

Synthesis Example 4.

Coupler No. 25

Synthesis of 2-(m-cyanophenyl)ureido-4-chloro-5-{α-(2,4-di-tert-pentylphenoxy)-tetradecaneamido}phenol

To a suspension of 18.9 g. of 2-amino-4-chloro-5-nitrophenol in 200 mℓ. of toluene was added a solution of 16 g. of m-cyanophenyl isocyanate in 100 mℓ. of toluene under stirring at room temperature. The resulting mixture was boiled under reflux for 1 hour. Thereafter, the reaction mixture was allowed to cool to room temperature. The crystalline substance thus separated was filtered, washed with hot toluene and then cold methanol and dried over sodium sulfate to give 33 g. of the product with mp 255-259°C.

A suspension of 3.3 g. of the so obtained 2-(m-cyanophenyl)ureido-4-chloro-5-nitrophenol in 200 mℓ. of tetrahydrofuran was subjected to catalytic reduction using palladium-carbon catalyst. After a theoretical volume of hydrogen was absorbed, 0.9 mℓ. of pyridine was added to the reaction mixture and a solution of 4.8 g. of 2-(2,4-di-tert-pentylphenoxy)tetradecanoyl chloride in 50 mℓ. of tetrahydrofuran was added thereto under stirring at room temperature. After completion of the addition, the reaction was continued for further 1 hour. The catalyst was filtered off, the filtrate was added to ice-water containing 10 mℓ. of conc. hydrochloric acid and extracted with ethyl acetate. The extract was washed with water, dried over sodium sulfate and concentrated under reduced pressure to leave an oily substance. The substance was purified by silica gel column chromatography and solidified with hexane to give 2.7 g. of the end product as a white solid with mp 185-188°C.

Analysis (%)

| | C | H | N | Cℓ |
|---|---|---|---|---|
| Calc'd: | 70.89 | 8.25 | 7.52 | 4.76 |
| Found : | 69.91 | 7.86 | 7.72 | 5.02 |

The cyan dye-forming coupler, which may be employed in this invention, can be used according to the methods and techniques commonly employed for conventional cyan dye-forming couplers. Typically the coupler can be blended with a silver halide emulsion and the emulsion coated onto a base to form a photographic element.

The photographic element may be monochromatic or multicolor one. In the case of a multicolor photographic element, the present cyan dye-forming coupler is usually incorporated into a red sensitive emulsion, but the element will also contain a non-sensitized emulsion or dye-image forming constituent units having photosensitivity to the three primary colors of the spectrum. Each constituent unit may comprise a monoemulsion layer or a multi-emulsion layer which has a photosensitivity to a certain region in spectrum. Element layers including the image-forming constituent layer may be arranged in any optional order as is well-known to those skilled in the art. Typical multicolor photographic elements comprise a cyan dye-forming image-forming constituent unit, said unit comprising at least one red sensitive silver halide emulsion layer containing at least one cyan dye-forming coupler (at least one of the couplers being a coupler of this invention) and a yellow dye image-forming constituent unit, said unit comprising at least one blue sensitive silver halide emulsion layer containing at least one magenta dye-forming coupler, both units being carried on a base. The element may further contain additional layers, for example a filter layer, an interlayer, a protective layer and a subbing layer.

The present coupler may be incorporated into an emulsion according to any well-known techniques. For instance, the present coupler alone or in combination with other ingredients may be dissolved in a high boiling organic solvent (boiling point of 175°C. or above) such as tricresyl phosphate or dibutyl phthalate or a low boiling organic solvent such as butyl acetate or butyl propionate alone or, if necessary, in combination therewith, the resulting solution is admixed with an aqueous solution of gelatin containing a surface active agent, the resulting mixture is then emulsified by a high speed rotary mixer or a colloid mixer and incorporated with a silver halide to prepare a silver halide emulsion which may be employed in this invention. The coupler of this invention is suitably employed in a range of usually about 0.07-0.7 mole, preferably 0.1-0.4 mole, per mole of silver halide of the silver halide emulsion.

As the silver halide which may be employed in the present silver halide emulsion, there may be used any silver halide commonly employed for a silver halide emulsion such as silver bromide, silver chloride, silver iodobromide, silver chlorobromide or silver chloroiodobromide.

A silver halide emulsion for the present invention may be prepared by various conventional methods such as the method disclosed, for example, in Japanese Patent Publication No. 46-7772: namely, a method for preparing a so-called conversion emulsion wherein a silver salt grain emulsion is formed, the said grain comprising at least partly a silver salt having a higher solubility than that of silver bromide, and then at least part of the said silver salt is converted to silver bromide or silver iodobromide, or a method for preparing the so-called Lippmann emulsion comprising silver halide grains having an average grain size of not more than 0.1 $\mu$.

Moreover, the silver halide emulsion may be chemically sensitized with a sulfur sensitizer such as allylthiocarbamide, thiourea or cystine, an active or inactive selenium sensitizer, a reduction sensitizer such as a stannous salt, a polyamine, a noble metal sensitizer such as a gold sensitizer, typically potassium aurithiocyanate, potassium chloroaurate or 2-aurosulfobenzothiazole methyl chloride, or a water-soluble ruthenium, rhodium or iridium, for example, salt sensitizer, typically ammonium chloropalladate, potassium chloroplatinate or sodium chloropalladate alone or in any combination.

The silver halide emulsion used herein may also contain a wide variety of well-known photographic additives, for example, those disclosed in "Research Disclosure", 1978, December, item 17643.

The silver halide emulsion may be spectrally sensitized using any suitable sensitizing dyes in order to afford photosensitivity to the sensitive wave length region required for a red sensitive emulsion. One or more spectrally sensitizing dyes may be used; for example those cyanine dyes, merocyanine dyes or complex cyanine dyes disclosed in U.S. Patents No. 2,269,234, No. 2,270,378, No. 2,442,710, No. 2,454,629, No. 2,776,280.

The color developing solution which may be employed in this invention preferably contains as a main ingredient an aromatic primary amine type color developing agent. Illustrative examples of such color developing agents are typically of the p-phenylenediamine type; for example, diethyl-p-phenylenediamine hydrochomethyl-p-phenylenediamine hydrochloride, dimethyl-p-phenylenediamine hydrochloride, 2-amino-5-diethyl-aminotoluene hydrochloride, 2-amino-5-(N-ethyl-N-dodecylamino)toluene, 2-amino-5-(N-ethyl-N-β-methanesulfonamidoethyl)aminotoluene sulfate, 4-(N-ethyl-N-β-methanesulfonamidoethylamino)aniline, 2-amino-5-(N-ethyl-N-β-methoxyethyl)aminotoluene and 4-(N-ethyl-N-β-hydroxyethylamino)aniline.

After development, conventional steps of bleaching for removal of silver or silver halide, fixing or bleach-fixing, washing and drying may be applied.

The following Examples are given to illustrate this invention further.

Example 1.

0.03 mole of each of the couplers indicated in the following Table 1 and the following control couplers A, B and C was added separately to a mixture of the same weight of dibutyl phthalate and 3 times the volume of ethyl acetate and the mixture was heated to 60°C. to form a complete solution. The solution was added to an aqueous solution of "Alkanol B"(alkylnaphthalene sulfonate, Registered Trade Mark of E.I. DuPont) and gelatin, the resulting mixture was emulsified by a colloid mill to prepare coupler dispersions of each coupler. Then, each coupler dispersion was added to a silver chlorobromide emulsion (20 mole % silver bromide; containing 0.1 mole silver) and the mixture was coated onto a polyethylene laminated paper and then dried to prepare 6 silver halide photosensitive materials for color photography having a stable coated film (Samples No. 1 to No. 6).

Control coupler A:

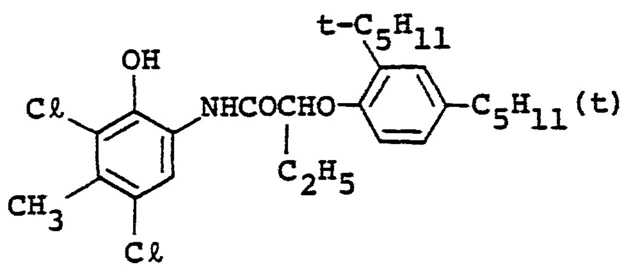

Control coupler B:

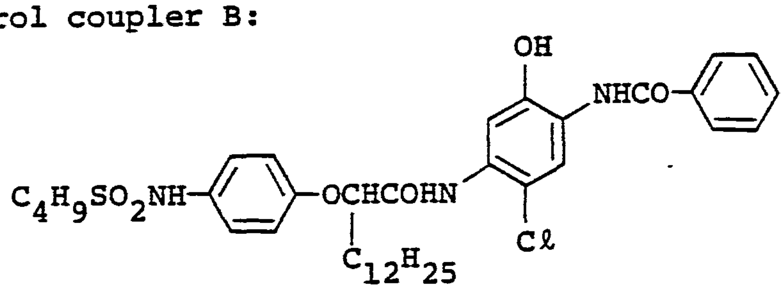

Control coupler C:

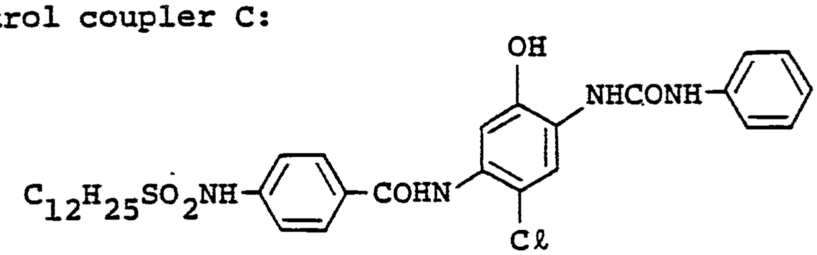

Each sample was subjected to wedge exposure according to a conventional method and then treated as stated hereunder. However, the color developing step was effected with two sorts of color developing compositions, namely color development (1) with benzyl-alcohol and color development (2) without benzyl alcohol.

(Treatment)

| Treatment step (30°C) | Treatment time |
|---|---|
| Color development | 3 min. 30 sec. |
| Bleach-fixing | 1 min. 30 sec. |
| Water washing | 2 min. |

Formulations for each step are given below.

(Color developing solution, composition 1)

| | |
|---|---|
| 4-Amino-3-methyl-N-ethyl-N-(β-methanesulfonamidoethyl)-aniline sulfate | 5.0 g. |
| Benzyl alcohol | 15.0 mℓ. |
| Sodium hexametaphosphate | 2.5 g. |
| Anhydrous sodium sulfite | 1.85 g. |
| Sodium bromide | 1.4 g. |
| Potassium bromide | 0.5 g. |
| Borax | 39.1 g. |

Water to make up 1 ℓ.

Adjusted to pH 10.30 with NaOH

(Color developing solution, composition 2) The same formulation as in the above composition 1 except that the benzyl alcohol was omitted.

(Bleach-fixing solution composition)

| | |
|---|---|
| Ethylenediaminetetraacetate iron ammonium complex | 50 g. |
| Ammonium sulfite (40% aqueous solution) | 50 mℓ. |
| Ammonium thiosulfate (70% aqueous solution) | 140 mℓ. |
| Aqueous ammonia (28% solution) | 20 mℓ. |
| Ethylenediaminetetraacetic acid | 4 g. |
| Water to make up 1 ℓ. | |

Each sample was assessed for its photographic characteristics. The results are summarized in Table 1, wherein relative sensitivity values are represented in terms of the maximum sensitivity value when treated with the color developing solution 1 taken as 100.

Table 1.

| Sample No. | Coupler applied | Color development 1 | | Color development 2 | |
|---|---|---|---|---|---|
| | | Relative sensitivity | Maximum density | Relative sensitivity | Maximum density |
| 1 | No. 6 | 98 | 2.22 | 70 | 1.77 |
| 2 | No. 8 | 100 | 2.24 | 75 | 1.80 |
| 3 | No. 17 | 98 | 2.20 | 71 | 1.80 |
| 4 | Control coupler A | 97 | 2.17 | 50 | 1.39 |
| 5 | Control coupler B | 94 | 1.90 | 61 | 1.45 |
| 6 | Control coupler C | 86 | 1.81 | 55 | 1.52 |

As can be seen from the above Table 1, the samples prepared from the present couplers show a good sensitivity and maximum density, irrespective of the presence or absence of benzyl alcohol.

Further, assessment of the color spectra has revealed that the dyes with the present coupler show an absorption maximum within a relatively longer wave length range of the red region and little absorption within the short wave length range and thus show excellent color purity.

Example 2.

The samples as prepared in the same manner as in the above Example 1 were tested for light fastness, heat fastness and moisture fastness of their dye images. The results are summarized in Table 2.

Table 2.

| Sample No. | Coupler applied | Color development 1 | | | Color development 2 | | |
|---|---|---|---|---|---|---|---|
| | | Light fastness | Heat fastness | Moisture fastness | Light fastness | Heat fastness | Moisture fastness |
| 7 | No. 6 | 90 | 98 | 96 | 91 | 97 | 95 |
| 8 | No. 8 | 88 | 96 | 97 | 87 | 96 | 96 |
| 9 | No. 17 | 85 | 97 | 96 | 84 | 96 | 96 |
| 10 | Control coupler A | 85 | 40 | 62 | 85 | 42 | 60 |
| 11 | Control coupler B | 65 | 90 | 91 | 55 | 95 | 90 |
| 12 | Control coupler C | 55 | 91 | 90 | 51 | 90 | 90 |

In the above Table 2, light fastness is represented in terms of the residual density of each image after exposure to a xenon fadeometer over 300 hours, the density before exposure being taken as 100; moisture fastness is represented in terms of the residual density after storage under a relative humidity of 70% over 3 weeks, the density before testing being taken as 100; and heat fastness is represented in terms of the residual density after storage at 77°C over 3 weeks, the density before testing being taken as 100, provided that the initial density is 1.0.

As is apparent from the above Table 2, the control coupler A possesses excellent light fastness, but not so good heat and moisture fastness, while control couplers B and C possess excellent heat and moisture fastness but not so good light fastness upon color development 2. In contrast, the present couplers Nos. 6, 8 and 17 are clearly excellent in every respect.

Example 3.

0.01 mole of each of the present couplers indicated in the following Table 3 and the above control couplers A and B and the following control coupler D was added (separately) to a mixture of the same weight of tricresyl phosphate and 3 times the volume of ethyl acetate and the resulting mixture was heated to 60°C. to form a complete solution. The solution was added to an aqueous solution of "Alkanol B" and gelatin, the resulting mixture was emulsified by a colloid mill to prepare coupler dispersions of each coupler.

Then, each coupler dispersion was added to a silver iodobromide emulsion (6 mole % silver iodide, containing 0.1 mole silver) and the mixture was coated onto a cellulose acetate film base and then dried to prepare 6 silver halide photosensitive materials for color photography having a stable coated film (Samples No. 13 to No. 18).

Control coupler D:

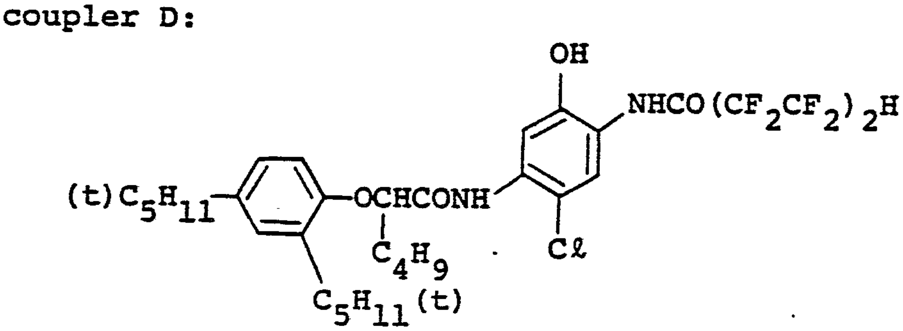

Each sample was subjected to wedge exposure according to a conventional method and then treated as stated hereunder.

(Treatment)

| Treatment step (33° C.) | Treatment time |
|---|---|
| Color development | 3 min. 15 sec. |
| Bleach | 6 min. 30 sec. |
| Water washing | 3 min. 15 sec. |
| Fixing | 6 min. 30 sec. |
| Water washing | 3 min. 15 sec. |
| Stabilization | 1 min. 30 sec. |

(Color developing solution, composition)

| | |
|---|---|
| 4-Amino-3-methyl-N-ethyl-N-(β-hydroxyethyl)aniline sulfate | 4.8 g. |
| Anhydrous sodium sulfate | 0.14 g. |
| Hydroxylamine 1/8 sulfate | 1.98 g. |
| Sulfuric acid | 0.74 g. |
| Anhydrous potassium carbonate | 28.85 g. |
| Anhydrous potassium hydrogencarbonate | 3.46 g. |
| Anhydrous potassium sulfite | 5.10 g. |
| Potassium bromide | 1.16 g. |
| Sodium chloride | 0.14 g. |
| Nitriloacetic acid trisodium salt | 1.20 g. |
| Potassium hydroxide | 1.48 g. |
| Water to make up 1 ℓ. | |

(Bleaching solution, composition)

| | |
|---|---|
| Ethylenediaminetetraacetato iron ammonium complex | 100 g. |
| Ethylenediaminetetraacetato di-ammonium salt | 10 g. |
| Ammonium bromide | 150 g. |
| Glacial acetic acid | 10 mℓ. |
| Water to make up 1 ℓ. | |

Adjusted to pH 6.0 with aqueous ammonia

(Fixer, composition)

| | |
|---|---|
| Ammonium thiosulfate | 175.0 g. |
| Anhydrous sodium sulfite | 8.6 g. |
| Sodium metasulfite | 2.3 g. |
| Water to make up 1 ℓ. | |
| Adjusted to pH 6.0 with acetic acid | |

(Stabilizer, composition)

| | |
|---|---|
| Formalin (37% aqueous solution) | 1.5 mℓ. |
| Konidax (manufactured by Konishiroku Photo Ind. Co., Ltd.) | 7.5 mℓ. |
| Water to make up 1 ℓ. | |

The produced cyan color images were assessed for photographic characteristics. The results are summarized in Table 3.

Table 3.

| Sample No. | Coupler applied | Relative sensitivity | Maximum density |
|---|---|---|---|
| 13 | No. 1 | 95 | 2.03 |
| 14 | No. 5 | 96 | 2.10 |
| 15 | No. 8 | 100 | 2.20 |
| 16 | Control coupler A | 92 | 1.66 |
| 17 | Control coupler B | 85 | 1.55 |
| 18 | Control coupler D | 80 | 1.48 |

As apparent from the above Table 3, the samples using the present coupler possess excellent sensitivity and color development.

Also, these samples containing couplers of the present invention as a result of spectral measurement, have been found to show an absorption maximum in a long wave length range of the red region and sharpness in a short wave length range and thus produce favourable dye images in color reproduction in the green region, as compared with the control couplers.

Example 4.

The samples No. 1 to No. 6 prepared in the above Example 1 were subjected to wedge exposure and then developed with the composition 1 in Example 1, except that a developing treatment was carried out with a bleach-fixing solution having the following composition to study discoloration of the cyan dye with an exhausted bleach-fixing solution.

(Bleach-fixing solution, composition)

| | |
|---|---|
| Ethylenediaminetetraacetato iron ammonium complex | 50 g. |
| Ammonium sulfite (40% solution) | 50 mℓ. |
| Ammonium thiosulfate (70% solution) | 140 mℓ. |
| Aqueous ammonia (28% solution) | 20 mℓ. |
| Ethylenediaminetetraacetic acid | 4 g. |
| Hydrosulfite | 5 g. |
| Water to make up 1 ℓ. | |

The sample thus treated was assessed for reflection density of the cyan dye. The results are summarized in Table 4. Dye residual rate at the maximum density is calculated from the following equation.

$$\text{Dye residual rate} = \frac{\text{Treatment with fresh bleach-fixing solution}}{\text{Treatment with exhausted bleach-fixing solution}} \times 100$$

**Table 4.**

| Coupler applied | Fresh BF* treatment | Exhausted BF* treatment | Dye residual rate |
|---|---|---|---|
| No. 6 | 2.20 | 2.18 | 99 |
| No. 8 | 2.22 | 2.18 | 98 |
| No. 17 | 2.18 | 2.16 | 99 |
| Control coupler A | 2.15 | 1.35 | 63 |
| Control coupler B | 1.98 | 1.92 | 97 |
| Control coupler C | 1.80 | 1.75 | 97 |

* BF = Bleach-fixing solution

It can be seen from Table 4 that the sample using the present couplers show less discoloration of cyan dye when treated with exhausted bleach-fixing solution..

Example 5.

Samples prepared in the same manner as in the above Example 1 were tested for light fastness, heat fastness and moisture fastness. The results are summarized in Table 5.

Table 5.

| Sample No. | Coupler applied | Color development 1 | | | Color development 2 | | |
|---|---|---|---|---|---|---|---|
| | | light fastness | heat fastness | moisture fastness | light fastness | heat fastness | moisture fastness |
| 19 | No. 20 | 85 | 98 | 97 | 86 | 98 | 98 |
| 20 | No. 21 | 86 | 98 | 98 | 87 | 98 | 98 |
| 21 | No. 25 | 86 | 98 | 97 | 86 | 97 | 97 |
| 22 | Control coupler A | 85 | 45 | 60 | 86 | 46 | 62 |
| 23 | Control coupler B | 61 | 95 | 93 | 57 | 96 | 95 |
| 24 | Control coupler C | 55 | 94 | 92 | 52 | 95 | 93 |

In the above Table 5, light fastness, moisture fastness and heat fastness are represented in the same manner as in the above Table 2.

As can be seen from the above Table 5, the control couplers A, B and C can be evaluated as in Table 2. On the other hand, the present couplers Nos. 20, 21 and 25 are clearly excellent in every respect.

Example 6.

The same procedures as in the above Example 3 were repeated except that the present couplers indicated in Table 6 were instead employed, thereby producing 6 silver halide photosensitive materials for color photography (Samples Nos. 25 to 30).

The samples were exposed and treated in the same manner as in the above Example 3.

The produced cyan color image was assessed for photographic characteristics. The results are summarized in Table 6.

Table 6.

| Sample No. | Coupler applied | Relative sensitivity | Maximum density |
|---|---|---|---|
| 25 | No. 24 | 98 | 2.05 |
| 26 | No. 28 | 100 | 2.20 |
| 27 | No. 40 | 100 | 2.09 |
| 28 | Control coupler A | 100 | 1.67 |
| 29 | Control coupler B | 93 | 1.68 |
| 30 | Control coupler D | 80 | 1.53 |

As is apparent from the above Table 6, the samples using the present couplers possess excellent sensitivity and color development.

Also, the samples using the couplers of the present invention as a result of spectral measurement, have been found to show an absorption maximum in a long wave length range of the red region and sharpness in a short wave length range and thus produce a favourable dye image in color reproduction in the green region, as compared with the control couplers.

Example 7.

The samples No. 19 to No. 24 as prepared in the above Example 5 were exposed, treated and tested for cyan dye maximum reflection density in the same manner as in the above Example 4. The results are summarized in Table 7.

Table 7.

| Coupler applied | Fresh BF treatment | Exhausted BF treatment | Dye residual rate |
|---|---|---|---|
| No. 20 | 2.19 | 2.19 | 100 |
| No. 21 | 2.17 | 2.15 | 99 |
| No. 25 | 2.17 | 2.17 | 100 |
| Control coupler A | 2.18 | 1.37 | 63 |
| Control coupler B | 1.90 | 1.84 | 97 |
| Control coupler C | 1.80 | 1.73 | 96 |

It can be seen from the Table 7 that the samples using the present couplers show less discoloration of cyan dye when treated with exhausted bleach-fixing solution.

Example 8.

Samples were obtained in the same manner as in Example 1 except that the couplers shown in Table 8 were used, and their photographic properties were measured according to the same procedures as in Example 1. The results are shown in Table 8.

Table 8.

| Sample No. | Coupler applied | Color development 1 | | Color development 2 | |
|---|---|---|---|---|---|
| | | Relative sensitivity | Maximum density | Relative sensitivity | Maximum density |
| 50 | 20 | 100 | 2.20 | 72 | 1.83 |
| 51 | 21 | 100 | 2.18 | 72 | 1.82 |
| 52 | 25 | 99 | 2.18 | 70 | 1.80 |

**Claims**

1. A silver halide photosensitive material suitable for color photography which comprises a phenol type cyan coupler having the formula

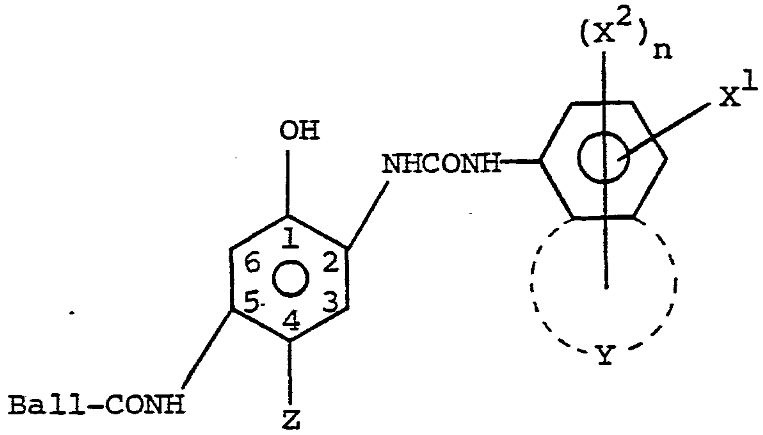

wherein

$X^1$ is -CN

$X^2$ is a halogen atom, a hydroxy group, a nitro group or an optionally substituted alkyl, alkylcarbonyloxy, arylcarbonyloxy, alkoxy, aryloxy, alkylcarbonyl or arylcarbonyl group;

Y, when present, is a group of non-metallic atoms which completes a 5-or 6-membered condensed ring;

Z is a hydrogen atom or a group removable upon coupling with an oxidation product of a color developing agent;

Ball is a ballast group; and

n is 0 or an integer from 1 to 4 inclusive such that, when n is 2 or more, each $X^2$ may be the same or different.

2. A photosensitive material according to Claim 1 wherein the phenylureido group substituted at the 2-position in the phenyl moiety has the formula

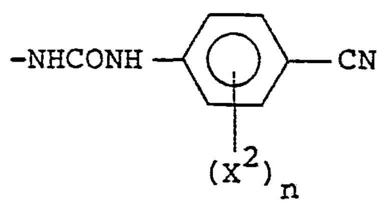

wherein $X^2$ is as defined in Claim 1 and n is an integer of 1 to 4 inclusive.

3. A photosensitive material according to claim 1 wherein the phenylureido group substituted at the 2-position in the phenyl moiety has the formula

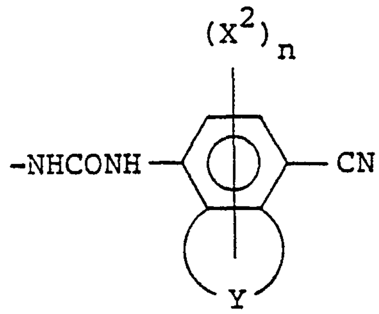

wherein Y and $X^2$ are as defined in Claim 1, and n is as defined in Claim 2.

4. A photosensitive material according to Claim 1 wherein said phenol type cyan coupler has the formula

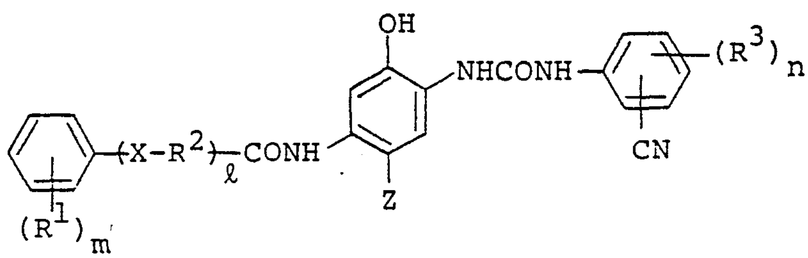

wherein

the -CN group is located at the 2-or 3-position to the ureido group;

X is an oxygen atom or a sulfur atom;

$R^2$ is a straight or branched alkylene group;

$R^3$ is a hydrogen atom, a halogen atom or an optionally substituted alkyl, alkylcarbonyloxy, arylcarbonyloxy, alkoxy, aryloxy, alkylcarbonyl or arylcarbonyl group;

$R^1$ is a hydrogen, a hydroxy group or an optionally substituted alkyl, aryl, heterocyclic, aralkyl, alkoxy, aryloxy, alkylcarbonyloxy, arylcarbonyloxy, carboxy, alkoxycarbonyl, aryloxycarbonyl, mercapto, alkylthio, arylthio, alkylsulfonyl, arylsulfonyl, alkylcarbonyl, arylcarbonyl, alkylcarbonylamino, arylcarbonylamino, sulfonamido, carbamoyl or sulfamoyl group;

n is 0 or an integer from 1 to 3;

m is an integer from 1 to 4; and

ℓ is 0 or 1, and Z is as defined in Claim 1.

5. A photosensitive material according to Claim 1, wherein the phenol type cyan coupler of formula I is one of couplers (1) to (42), (71), (73), (76), and -(78), specifically identified herein.

6. A photosensitive material according to any one of the preceding claims which is in the form of a silver halide emulsion.

7. A photosensitive element which comprises an emulsion as claimed in Claim 6 on a base.

8. A phenol type cyan coupler of formula I as defined in any one of Claims 1 to 5.

European Patent Office

# EUROPEAN SEARCH REPORT

Application number

EP 86 10 4642

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 028 099 (EASTMAN-KODAK)<br>* Claims *<br>----- | 1 | G 03 C 7/34 //<br>C 07 C 127/19 |
| | | | **TECHNICAL FIELDS SEARCHED (Int. Cl.4)** |
| | | | G 03 C 7 |

The present search report has been drawn up for all claims

| Place of search | Date of completion of the search | Examiner |
|---|---|---|
| THE HAGUE | 20-08-1986 | PHILOSOPH L.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82